# EUROPEAN PATENT APPLICATION

(11) **EP 4 378 305 A1**
(43) Date of publication of application: **05.06.2024**
(21) Application number: 22306777.8
(22) Date of filing: 02.12.2022
(51) Int. Cl.: A01H 5/02, A01H 6/00, A01H 1/00

(54) **METHOD FOR DETECTING CROSS-COMPATIBILITY AMONG OLIVE TREES**

(71) Applicant: Université Toulouse III-Paul Sabatier, 31062 Toulouse Cedex 9 (FR); Centre National de la Recherche Scientifique, 75016 Paris (FR); Institut de Recherche pour le Développement, 13572 Marseille Cedex 02 (FR); France Olive, 13626 Aix en Provence Cedex 1 (FR)
(72) Inventor: BESNARD, Guillaume, 31062 TOULOUSE CEDEX 9 (FR); RAIMONDEAU, Pauline, 31400 TOULOUSE (FR)
(74) Representative: Lavoix

(57) **Abstract**

The present invention concerns a method for determining the self-incompatibility genotype or phenotype of an olive tree, thereby enabling determining cross-compatibility among olive trees and adapting planting thereof in an orchard, for instance to optimize pollination, a major issue in olive production .

## Description

The present invention concerns a method for determining the self-incompatibility genotype or phenotype of an olive tree, thereby enabling determining cross-compatibility among olive trees and adapting planting thereof in an orchard, for instance to optimize their pollination, a major issue in olive production.

### BACKGROUND

Olive (*Olea europaea* L., Oleaceae) is a major fruit crop that accompanied the emergence of first Mediterranean civilizations. Today, several hundreds of varieties are grown around the world to produce high-quality fruits for oil and for table consumption, but most of them have a very restricted distribution. Controlling olive production depends on several factors, and in particular an efficient pollination, that may be not optimal in some conditions. Indeed, most of olive cultivars are fully self-incompatible, and a very few are able to produce some selfings (Alagna *et al.,* 2019, *Front Plant Sci* 10: 725). In addition, cultivated olives are vegetatively propagated (clones), and consequently the number of genotypes can be very limited in orchards and surrounding habitats, particularly in regions with no wild olives around.

Self-incompatibility (SI) is the most common mechanism to promote outbreeding in flowering plants. It consists of pollen-pistil recognition systems that prevent self-fertilization but also cross-fertilization between individuals sharing the same compatibility type. To date, few SI systems have been characterized at the molecular level. All vary in their precise functioning, but they are generally encoded by a single genomic region, the S-locus, containing several genes, encoding pollen and pistil components.

In olive (*Olea europaea* L., Oleaceae), decisive progress has been made in the past few years towards the elucidation of the SI mechanism. First, examinations of cross-compatibilities revealed the existence of a peculiar homomorphic sporophytic SI system with only two incompatibility groups (Saumitou-Laprade et al., 2017, Evol. Appl. 10: 867-880; Besnard et al., 2020, Ecol. Evol. 10: 1876-1888). To explain this configuration, it has been hypothesized that two alleles exist at the so-called diallelic self-incompatibility (DSI) locus, *S* and *s*, with *S* dominant over *s,* and with only two genotypic combinations (*Ss* and ss), corresponding to G1 and G2 incompatibility groups, respectively (Saumitou-Laprade et al., 2010, Science 327: 1648-1650).

Currently, phenotyping the cross-compatibility of varieties requires testing (either bag crosses which are not very reliable; or stigma tests under the microscope). These tests obviously require the plant to be in full bloom, which is not the case most of the year. In addition, paternity tests can also be used to determine compatibility groups, but they are relatively long and very expensive. The development of a diagnostic test diagnostic test is therefore an important step forward in facilitating the phenotyping of DSI compatibility of crossing any olive tree at any stage (e.g. seedling).

Recently, Mariotti et al. (2020, Front. Plant Sci. 10: 1760) have developed a marker linked to the DSI locus, but the inventors found that this tool failed to recognize compatibility groups in an olive collection, both on cultivated and wild olives.

Using a combination of already available and newly generated genomics data, the inventors were able to refine the genomic mapping of the olive S-locus and found that the previous candidate region, identified using linkage in a segregating population of cultivated olive (Mariotti et al., 2020, Front. Plant Sci. 10: 1760), was erroneously associated with self-incompatibility. This probably was the consequence of an incomplete assembly of the DSI region that hampered a correct identification of the locus. The inventors' work revealed that homomorphic di-allelic self-incompatibility system in olive is governed by a hemizygous region on chromosome 18. The dominant *S*-haplotype harbors a 0.7-Mb region that is present in only one of the two incompatibility groups, specifically in *Ss* individuals (or group G1).

### SUMMARY OF THE INVENTION

The invention relates to a method for determining the self-incompatibility genotype of an olive tree comprising:
a) detecting the presence (allele *S*) or absence (allele *s*) of a 0.7 Mb insertion-deletion (indel) sequence in its chromosomes 18;
b) determining the self-incompatibility genotype of the olive tree as being either *Ss* if the 0.7-Mb indel sequence is present at the hemizygous state in one chromosome 18, or ss if the 0.7-Mb indel sequence is absent in both chromosomes 18;
wherein allele *S* is dominant over allele s, and genotypes *Ss* and ss constitute the two inter-compatible genotypic combinations of the diallelic self-incompatibility (DSI) system in olive tree.

The invention further relates to a method for determining the self-incompatibility phenotype of an olive tree comprising:
a) detecting the presence (allele *S*) or absence (allele *s*) of a 0.7 Mb insertion-deletion (indel) sequence in its chromosomes 18;
b) determining the self-incompatibility phenotype of the olive tree as being either self-incompatibility group G1 if the 0.7-Mb indel sequence is present at the hemizygous state in one chromosome 18, or self-incompatibility group G2 if the 0.7-Mb indel sequence is absent in both chromosomes 18;
wherein olive trees from group G1 cannot cross-fertilize each others, and olive trees from group G2 cannot cross-fertilize each others.

Also provided is a method for determining cross-compatibility between at least two olive trees, comprising:
a) determining the self-incompatibility phenotype or genotype of at least two olive trees by a method according to the invention;
b) identifying the at least two olive trees as cross-compatible if they have different self-incompatibility phenotypes (G1 and G2) or genotypes (*Ss* and *ss*)*,* and identifying the two olive trees as cross-incompatible if they have the same self-incompatibility phenotype (either G1 and G1, or G2 and G2) or genotype (either *Ss* and *Ss,* or ss and ss).

In another aspect, the invention relates to a method of planting olive trees in an orchard comprising:
a) determining the self-incompatibility genotype or phenotype of olive trees by a method according to the invention;
b) selecting olive trees of the G1 and G2 phenotypes, or olive trees of the *Ss* and ss genotypes;
planting olive trees in the orchard with a spatial repartition of olive trees defined based on the G1 and G2 phenotypes, or the Ss and ss genotypes of the olive trees.

The invention further relates to a method of optimizing olive production comprising:
a) planting olive trees in an orchard by a method according to the invention;
b) cultivating the planted olive trees under conditions and for a time sufficient for the olive trees to produce olives, and harvesting olives on the cultivated olive trees.

In still another aspect, the invention relates to a kit comprising:
i) A forward primer comprising sequence SEQ ID NO:9, a first reverse primer comprising sequence SEQ ID NO:11, and a second reverse primer comprising sequence SEQ ID NO:12 or sequence SEQ ID NO:13;
ii) A forward primer comprising sequence SEQ ID NO:14 and a reverse primer comprising sequence SEQ ID NO:16; or
iii) A forward primer comprising sequence SEQ ID NO:17 and a reverse primer comprising sequence SEQ ID NO:18.

Also provided is a method of performing PCR amplification, comprising:
a) Hybridizing a sample of genomic DNA of olive tree with a set of primers comprising:
   [1] A forward primer comprising sequence SEQ ID NO:9, a first reverse primer comprising sequence SEQ ID NO:11, and a second reverse primer comprising sequence SEQ ID NO:12 or sequence SEQ ID NO:13;
   [2] A forward primer comprising sequence SEQ ID NO:14 and a reverse primer comprising sequence SEQ ID NO:16; or
   [3] A forward primer comprising sequence SEQ ID NO:17 and a reverse primer comprising sequence SEQ ID NO:18; and
b) Performing PCR amplification of said genomic DNA.

### DETAILED DESCRIPTION

Controlling olive production depends on an efficient pollination that may be not optimal in some conditions, especially because most of olive cultivars are fully self-incompatible and the number of cultivated genotypes is often limited in orchards. Cross-compatibility among olive trees is mainly controlled by a homomorphic di-allelic self-incompatibility system (DSI) that produces two interfertile groups [G1 (*Ss*) and G2 (*ss*)] that need to be properly assorted in orchards to optimize pollination.

The inventors have demonstrated that G1 individuals all harbor a hemizygous 0.7-Mb region corresponding to allele *S.*

A PCR-based test was thus developed by the inventors to determine the group of compatibility of any olive genotype. Primers were defined in conserved regions at proximity of the two insertion sites of the hemizygous region in order to simultaneously amplify both *S* and *s* alleles. The test was validated on a panel of cultivated and wild olives (130 trees belonging to three diploid olive subspecies) that were previously phenotyped for the cross-compatibility group. The strong reliability and robustness of this test were thus demonstrated on a wide panel of olive accessions ensuring its potential use for rapidly determining cross-compatibility groups in breeding programs and research studies.

### Method for determining the self-incompatibility genotype or phenotype of an olive tree

The method for determining the self-incompatibility genotype of an olive tree comprises detecting the presence (allele *S*) or absence (allele *s*) of a 0.7 Mb insertion-deletion (indel) sequence in the chromosomes 18 of the olive tree. Based on said detection of the 0.7-Mb indel sequence, the self-incompatibility genotype of the olive tree is determined as being either *Ss* if the 0.7-Mb indel sequence is present at the hemizygous state in one chromosome 18, or *ss* if the 0.7-Mb indel sequence is absent in both chromosomes 18.

Olive tree is a diploid species. Allele *S* is dominant over allele *s.* Genotypes *Ss* and ss constitute the two inter-compatible genotypic combinations of the diallelic self-incompatibility (DSI) system in olive tree, and correspond to G1 and G2 incompatibility groups, respectively. While olive trees from group G1 cannot cross-fertilize each others, and olive trees from group G2 cannot cross-fertilize each others, G1 × G2 crosses generate progenies.

Accordingly, based on said detection of the 0.7-Mb indel sequence, the self-incompatibility phenotype of the olive tree can also be determined as being either self-incompatibility group G1 if the 0.7-Mb indel sequence is present at the hemizygous state in one chromosome 18, or self-incompatibility group G2 if the 0.7-Mb indel sequence is absent in both chromosomes 18.

In the Mediterranean Basin, Subtropical Africa and southern Asia, three diploid olive subspecies are currently recognized (Green, 2002. Kew Bull. 57: 91-140): *Olea europaea* subsp. *europaea* (Mediterranean olive), *Olea europaea.* subsp. *laperrinei* (Laperrine's olive), and *Olea europaea.* subsp. *cuspidata* (African olive). Accordingly, in some embodiments, the olive tree in the frame of the invention is *Olea europaea* subsp. *europaea, Olea europaea.* subsp. *laperrinei or Olea europaea.* subsp. *cuspidata,* or a hybrid thereof.

The 0.7-Mb indel sequence localizes at positions 1,658,780 to 2,393,593 in haplocontig h1tg000037I of a G1 Laperrine's olive genome (individual 'Adjelella_9_S4'), and is present at positions 100,501 to 835,222 of sequence SEQ ID NO:19. As it will be appreciated by those skilled in the art, variants of the 0.7-Mb indel sequence SEQ ID NO:19 may be found in other Laperrine's olive individuals, or in other *Olea europaea* subspecies.

According to an embodiment, the 0.7-Mb indel sequence in chromosome 18 of the olive tree comprises, or consists of, nucleotides from position 100501 to position 835222 of sequence SEQ ID NO:19, or a sequence at least 75%, 80%, 85%, 90%, or 95% identical to SEQ ID NO:19. In the frame of the invention, the sequence at least 75% identical to SEQ ID NO:19 is a sequence that conditions self-incompatibility genotype or phenotype of an olive tree.

As used herein, determining the percentage of identity of query sequence with SEQ ID NO:19 is to be performed by pairwise global sequence alignment, for instance by Needleman-Wunsch global alignment using EMBOSS needle and default value (Gap Open Penalty =10, Gap Extend Penalty = 0.5).

Detecting the presence, at the hemizygous state, of the 0.7-Mb indel sequence in one chromosome 18 of the olive tree may performed by detecting the 5' or 3' insertion junction of the 0.7-Mb indel sequence in chromosome 18, or by detecting gene(s) that are only present in the 0.7-Mb indel sequence.

Seventeen genes were identified as specific to the 0.7-Mb S-specific region (G1 individuals) and are disclosed in Table 4 of the instant application. These include in particular genes g33210.t1 (comprising positions 780,191 to 783,682 of SEQ ID NO:19, or a sequence at least 75%, 80%, 85%, 90%, or 95% identical thereto), g33223.t1 (comprising positions 519496 to 520839 of SEQ ID NO:19, or a sequence at least 75%, 80%, 85%, 90%, or 95% identical thereto), g33231.t1 (comprising positions 652,393 to 655,078 of SEQ ID NO:19, or a sequence at least 75%, 80%, 85%, 90%, or 95% identical thereto), and g33233.t1 (comprising positions 668,762 to 670,268 of SEQ ID NO:19, or a sequence at least 75%, 80%, 85%, 90%, or 95% identical thereto). A predicted sequence of g33233.t1 gene mRNA for *Olea_europaea_*subsp*. europaea* var*._sylvestris* is available in Genbank under accession number XM_023018078 (version XM_023018078.1), it encodes a gibberellin 2-beta-dioxygenase-like protein.

According to some embodiments, detecting the presence, at the hemizygous state, of the 0.7-Mb indel sequence in one chromosome 18 of the olive tree is performed by:
i) Amplifying a region of at least 12, preferably at least 15, 20, 25, 30, or 35 contiguous nucleotides of chromosome 18 that comprises at least nucleotides at positions 422-423 of SEQ ID NO: 1 or SEQ ID NO:2. Preferably, a region that comprises nucleotides at positions 386-617 of SEQ ID NO:1 or nucleotides at positions 386-652 of SEQ ID NO:2 is amplified; or
ii) Amplifying a region of chromosome 18 that comprises nucleotides at positions 505-623 of SEQ ID NO: 3 or SEQ ID NO: 4; or
iii) detecting presence of at least one gene specific to the 0.7-Mb *S*-specific region, as disclosed above, in particular g33233.t1 gene (which is a hallmark of G1 individuals).

Conversely, detecting the absence of the 0.7-Mb indel sequence in chromosome 18 of the olive tree may be performed by detecting absence of at least one gene specific to the 0.7-Mb *S*-specific region, as disclosed above, in particular absence of gene g33233.t1.

Detection of the presence or absence of at least one gene specific to the 0.7-Mb *S-*specific region may also be performed by amplification of a segment of chromosome 18 that encompass at least a fragment of said at least one gene specific to the 0.7-Mb *S-*specific region, when the 0.7-Mb indel sequence is present in chromosome 18.

"Amplification" or "amplifying" refers to any known procedure for obtaining multiple copies of a target nucleic acid sequence or its complement or fragments thereof. The multiple copies may be referred to as amplicons or amplification products. Known amplification methods include both thermal cycling and isothermal amplification methods. Polymerase chain reaction (PCR), ligase chain reaction (LCR), strand-displacement amplification (SDA), and transcription-mediated or transcription-associated amplification are non-limiting examples of nucleic acid amplification methods. Replicase-mediated amplification uses self-replicating RNA molecules, and a replicase. PCR amplification uses a DNA polymerase, pairs of primers that are selected so as to enable specific amplification of a target nucleic acid sequence, and thermal cycling to synthesize multiple copies of two complementary strands of dsDNA or from a cDNA. LCR amplification uses four or more different oligonucleotides to amplify a target and its complementary strand by using multiple cycles of hybridization, ligation, and denaturation. SDA uses a primer that contains a recognition site for a restriction endonuclease and an endonuclease that nicks one strand of a hemi-modified DNA duplex that includes the target sequence, whereby amplification occurs in a series of primer extension and strand displacement steps.

According to an embodiment, amplification is performed by PCR.

A "primer sequence" is typically a short single-stranded nucleic acid, of between 10 to 50 nucleotides in length, designed to perfectly or almost perfectly match a nucleic acid of interest DNA or RNA), to be captured and then amplified by typically PCR or reverse transcribed and then amplified by typically RT-PCR. The primer sequences are "specific" to the nucleic acids they hybridize to, i.e. they preferably hybridize under stringency hybridization conditions, more preferably under high stringency hybridization conditions, or are complementary to or almost complementary to the nucleic acids they hybridize to, also called target sequence.

Typically, the primer sequence serves as a starting point for nucleic acid synthesis, allowing polymerase enzymes such as nucleic acid polymerase to extend the primer sequence and replicate the complementary strand. A primer sequence may be complementary to and hybridize to a target nucleic acid. In some embodiments, a primer sequence is a synthetic primer sequence. In some embodiments, a primer sequence is a non-naturally-occurring primer sequence. A primer sequence typically has a length of 10 to 50 nucleotides. For example, a primer sequence may have a length of 10 to 40, 10 to 30, 10 to 20, 25 to 50, 15 to 40, 15 to 30, 20 to 50, 20 to 40, or 20 to 30 nucleotides. In some embodiments, a primer sequence has a length of 18 to 24 nucleotides.

According to an embodiment, the method detects a sequence encompassing the 5' insertion junction of the 0.7-Mb indel sequence in chromosome 18 (e.g. DSI_A locus in following example 3). The method may then comprises:
a) detecting the presence, at the hemizygous state, or absence of a 0.7- Mb indel sequence in chromosomes 18 of an olive tree by PCR amplification using a forward primer comprising sequence SEQ ID NO:9, a first reverse primer comprising sequence SEQ ID NO:11, and a second reverse primer comprising sequence SEQ ID NO:12 or sequence SEQ ID NO:13; and
b) identifying the olive tree as having a genotype *Ss,* or phenotype G1, if the size of one of the amplicons is in the range 290 to 340 bp, in particular 298 to 332 bp, or identifying the olive tree as having a genotype *ss,* or phenotype G2, if the size of the amplicons is only 186 bp when the pair of reverse primers comprises SEQ ID NO: 11 and SEQ ID NO: 13, or only in the range 430 to 460 bp, in particular 438 to 452 bp, when the pair of reverse primers comprises SEQ ID NO: 11 and SEQ ID NO:12.

According to another embodiment, the method detects a region specifically found at the 3' end of the 0.7-Mb indel sequence in chromosome 18 (e.g. DSI_B locus in following example 3). The method may then comprises:
a) detecting the presence, at the hemizygous state, or absence of a 0.7-Mb indel sequence in chromosomes 18 of an olive tree by PCR amplification using a forward primer comprising sequence SEQ ID NO:14, in particular comprising sequence SEQ ID NO:15; and a reverse primer comprising sequence SEQ ID NO:16;
b) identifying the olive tree as having a genotype Ss, or phenotype G1, if the size of one of the amplicons is 185 bp, or identifying the olive tree as having a genotype ss, or phenotype G2, if the size of the amplicons is only at 190-191 bp.

According to another embodiment, the method detects the presence or absence of g33233.t1 gene in chromosomes 18 of the olive tree. The method may then comprises:
a) detecting the presence, at the hemizygous state, or absence of a 0.7-Mb indel sequence in chromosomes 18 of an olive tree by PCR amplification using a forward primer comprising sequence SEQ ID NO:17 and a reverse primer comprising sequence SEQ ID NO:18;
b) identifying the olive tree as having a genotype Ss, or phenotype G1, if the size an amplicon is 168 bp, or identifying the olive tree as having a genotype ss, or phenotype G2, if the PCR amplification produces no amplicon.

Suitable thermal cycling conditions for PCR amplification are disclosed in following example 3. These include in particular:
i) 2 min at 94°C, followed by 25 cycles of 30 s at 94°C, 45 s at 56°C, and 1 min at 72°C, and then by 10 cycles of 30 s at 94°C, 45 s at 51.5°C, and 45 s at 72°C, and then 20-min extension at 72°C for detection of DSI-A or DSI-B; or
ii) for 2 min at 94°C, followed by 35 cycles of 30 s at 94°C, 45 s at 56°C, and 1 min at 72°C, and then 20-min extension at 72°C for detection of g33233.t1.

The cross-compatibility of two (or more) olive trees can be determined based on the known self-incompatibility genotype or phenotype of each of the two (or more) olive trees.

Accordingly, a method is further provided for determining cross-compatibility between at least two olive trees (in particular two cultivars), comprising:
a) determining the self-incompatibility phenotype or genotype of at least two olive trees by a method of the invention;
b) identifying the at least two olive trees as cross-compatible if they have different self-incompatibility phenotypes (G1 and G2) or genotypes (Ss and ss), and identifying the two olive trees as cross-incompatible if they have the same self-incompatibility phenotype (either G1 and G1, or G2 and G2) or genotype (either *Ss* and *Ss,* or ss and ss).

### Method of planting olive trees

The knowledge of the cross-compatibility among cultivars enables to design crosses in olive breeding programs effectively. It also enables modulating the spatial repartition of olive trees, e.g. in order to maximize pollination between cross-compatible olive trees.

A method of planting olive trees in an orchard is therefore provided that comprises:
a) determining the self-incompatibility genotype or phenotype of olive trees by a method of the invention;
b) selecting olive trees of the G1 and G2 phenotypes, or olive trees of the *Ss* and ss genotypes;
c) planting olive trees in the orchard with a spatial repartition of olive trees defined based on the G1 and G2 phenotypes, or the *Ss* and *ss* genotypes of the olive trees.

In the method of the invention, all or part of the olive trees subjected to analysis are to be planted in the orchard. In some embodiments, part of the olive trees subjected to analysis are already planted in the orchard, and further planting olive trees in the orchard is performed to improve the spatial repartition of G1 and G2 olive trees, or the *Ss* and *ss* olive trees, in the orchard.

The spatial repartition may be for instance a homogenous repartition of G1/Ss and G2/ss olive trees in the orchard.

A method of optimizing (e.g. increasing) olive production may thus comprise:
c) planting olive trees (in particular young olive trees), considering their cross-compatibility to favor an optimal pollination in the orchard, by a method of the invention;
d) cultivating the planted olive trees under conditions and for a time sufficient for the olive trees to produce olives, and harvesting olives on the cultivated olive trees.

The olive trees are preferably *Olea europaea* subsp. *europaea* (Mediterranean olive), *O. e.* subsp. *laperrinei* (Laperrine's olive), or *O. e.* subsp. *cuspidata* (African olive), or a hybrid thereof.

### Kit and PCR method

The invention further relates to a kit comprising:
i) A forward primer comprising sequence SEQ ID NO:9, a first reverse primer comprising sequence SEQ ID NO:11, and a second reverse primer comprising sequence SEQ ID NO:12 or sequence SEQ ID NO:13;
ii) A forward primer comprising sequence SEQ ID NO:14 and a reverse primer comprising sequence SEQ ID NO:16; or
iii) A forward primer comprising sequence SEQ ID NO:17 and a reverse primer comprising sequence SEQ ID NO:18.

Said kit can be used for the determination of the self-incompatibility genotype or phenotype of an olive tree.

A method of performing PCR amplification is provided, comprising:
a) Hybridizing a sample of genomic DNA of olive tree with a set of primers comprising:
   [1] A forward primer comprising sequence SEQ ID NO:9, a first reverse primer comprising sequence SEQ ID NO:11, and a second reverse primer comprising sequence SEQ ID NO:12 or sequence SEQ ID NO:13;
   [2] A forward primer comprising sequence SEQ ID NO:14 and a reverse primer comprising sequence SEQ ID NO:16; or
   [3] A forward primer comprising sequence SEQ ID NO:17 and a reverse primer comprising sequence SEQ ID NO:18; and
b) Performing PCR amplification of said genomic DNA.

In said method of PCR amplification, the sample of genomic DNA of olive tree preferably comprises genomic DNA of chromosomes 18, or of a fragment thereof that includes at least positions 100,501 to 835,222 of sequence SEQ ID NO:19, or a sequence at least 75%%, 80%, 85%, 90%, or 95% identical to positions 100501 to 835222 of SEQ ID NO:19.

Suitable thermal cycling conditions for PCR amplification are disclosed in following example 3. These include in particular:
i) 2 min at 94°C, followed by 25 cycles of 30 s at 94°C, 45 s at 56°C, and 1 min at 72°C, and then by 10 cycles of 30 s at 94°C, 45 s at 51.5°C, and 45 s at 72°C, and then 20-min extension at 72°C for the set of primers according to [1] or [2]; or
ii) for 2 min at 94°C, followed by 35 cycles of 30 s at 94°C, 45 s at 56°C, and 1 min at 72°C, and then 20-min extension at 72°C for the set of primers according to [3].

Throughout the instant application, the term "and/or" is a grammatical conjunction that is to be interpreted as encompassing that one or more of the cases it connects may occur. For example, the wording "error detection and/or correction " in the phrase "the sequences allow for error detection and/or correction" indicates that the sequences may allow for error detection and the sequence may allow for error correction or the sequences may allow for error detection or the sequence may allow for error correction.

As used in this specification and the appended claims, the singular forms "a," "an" and "the" include plural references, such as a plurality of the object referred to, unless the content clearly dictates otherwise.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (*i.e.* "consisting of").

The invention will be further illustrated in view of the following figures and examples.

### DESCRIPTION OF THE SEQUENCES

SEQ ID NO:1: Allele S*_europaea* [Talambote; DSI-A: allele_S_298].
SEQ ID NO: 2: Allele S*_laperrinei* [Adjelella_9_S4; DSI-A: allele_S_332].
SEQ ID NO:3: Allele S*_europaea* [Talambote; DSI-B: allele_S_185].
SEQ ID NO:4: Allele S_*laperrinei* [Adjelella_9_S4; DSI-B: allele_S_185].
SEQ ID NO:5: Allele s*_europaea* [Talambote; DSI-A: allele_s_446].
SEQ ID NO:6: Allele s*_laperrinei* [Adjelella_9_S4; DSI-A: allele_s_442].
SEQ ID NO:7: Allele s*_europaea* [Talambote; DSI-B: allele s_191].
SEQ ID NO: 8: Allele s*_laperrinei* [Adjelella_9_S4; DSI-B: allele_s_191].
SEQ ID NO: 9: forward primer S/s_A (without M13 tail).
SEQ ID NO: 10: forward primer S/s_A (with M13 tail).
SEQ ID NO: 11: reverse primer S_A.
SEQ ID NO: 12: reverse primer s_A_(1).
SEQ ID NO: 13: reverse primer s_A(2).
SEQ ID NO: 14: forward primer S/s_B (without M13 tail).
SEQ ID NO: 15: forward primer S/s_B (with M13 tail).
SEQ ID NO: 16: reverse primer S/s_B.
SEQ ID NO: 17: forward primer g33233.
SEQ ID NO: 18: reverse primer g33233.
SEQ ID NO:19: 0.7-Mb indel sequence present in chromosome 18 of G1 Laperrine's olive genome (individual 'Adjelella_9_S4'), together with 100.5 kb long 5' and 3' flanking sequences.

### FIGURES

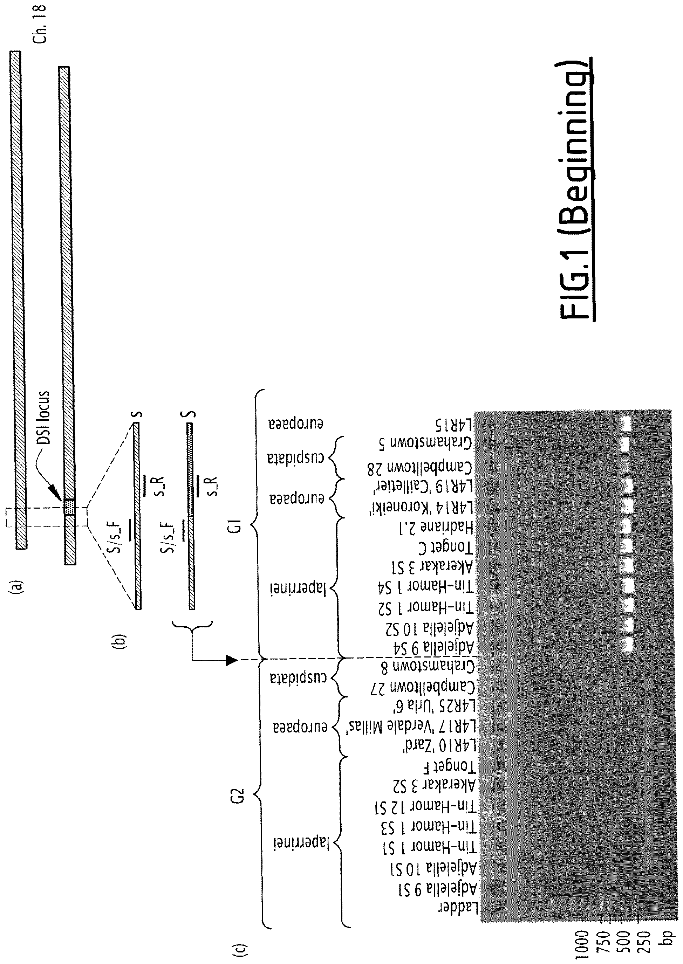

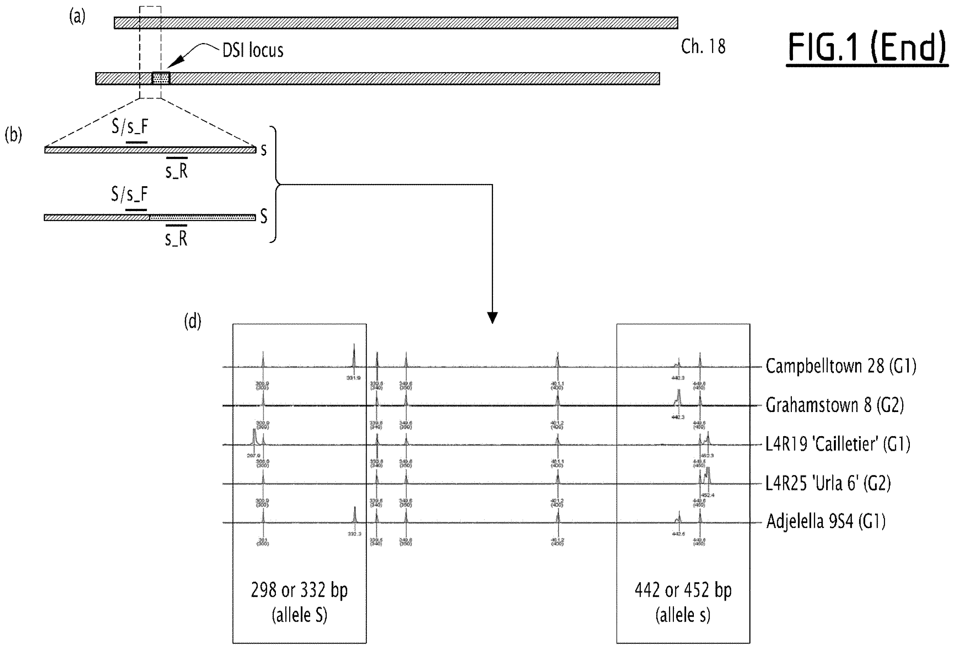

**Figure 1****:** General approach to develop a PCR test aiming at determining the cross-compatibility group in olive (here for marker DSI-A). **(a)** Schematic representation of the pair of chromosomes 18 in a G1 individual (expected to harbor the Ss genotype at the DSI locus. The DSI region (in yellow) is hemizygous on ca. 730 kb and is present only in G1 individuals. It thus corresponds to allele *S,* while the absence of this region corresponds to allele s. **(b)** On the 5' extremity covering the hemizygous region insertion site, three primers were defined in short conserved regions of *S* and/or *s* alleles. S/s_A_F was defined in a conserved region on both *S* and *s* alleles (less than 40 bp before the insertion of the hemizygous region), while one reverse primer was defined specifically for each *S* and s alleles. **(c)** Electrophoresis (on 2% agarose) of the PCR product obtained with S/s_A_For and S_A_Rev on 12 G2 and 12 G1 trees belonging to subspecies *laperrinei, europaea* and *cuspidata.* A fragment of 298 or 332 bp is revealed only in all G1 individuals (for *europaea* or *laperrinei-cuspidata,* respectively), while no amplification is observed in G2 individuals. **(d)** Chromatograms of the PCR product of five individuals after the simultaneous amplification of both *Sand* s alleles in the same reaction with S/s_A_For, s_(1)A_Rev, and S_A_Rev. One *S* allele is detected only in G1 individuals at an expected size of 298 or 332 bp, whereas s alleles are detected in all individuals at 442 to 452 bp.

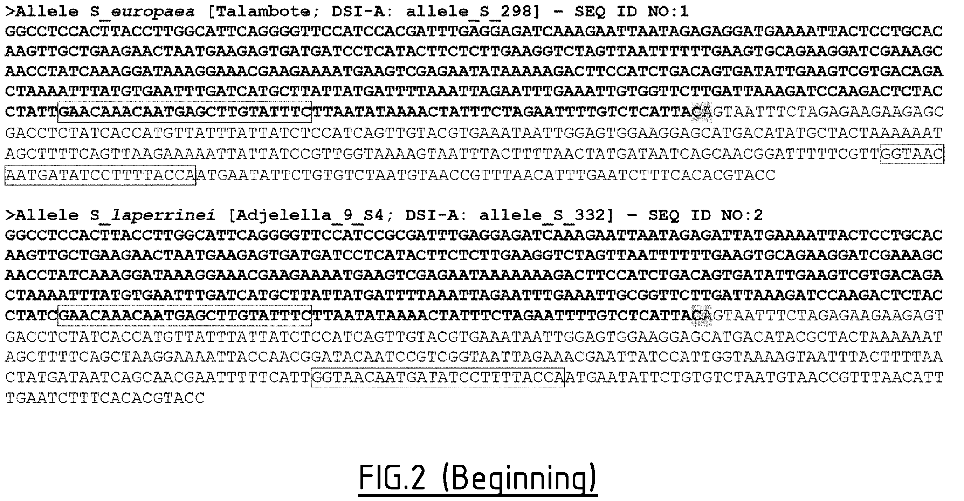

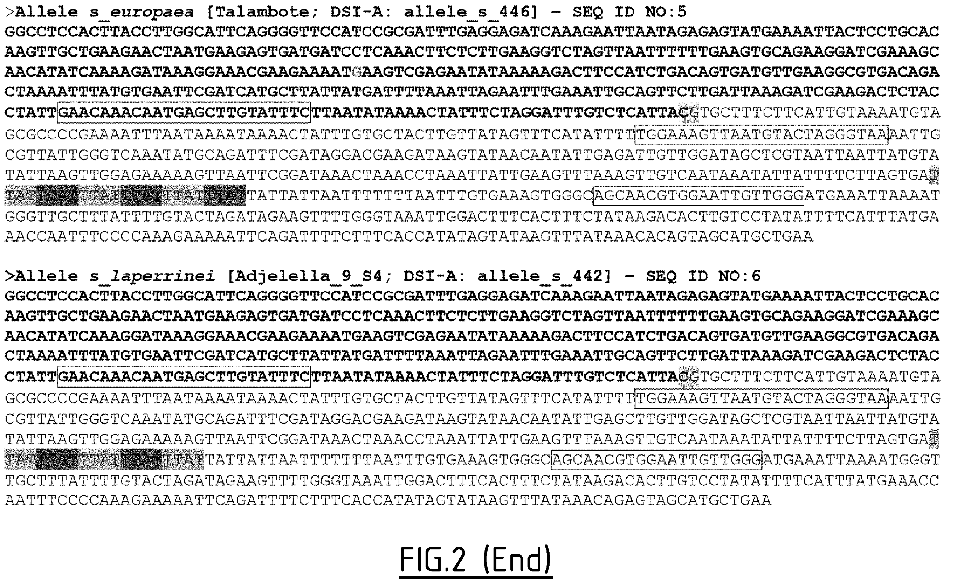

**Figure 2****:** DNA segments of S and s alleles at the two insertion sites of the hemizygous DSI region [Junction DSI-A]. Sequences were isolated from two G1 individuals belonging to subspecies *europaea* and *laperrinei.* Location of primers is represented by frames, and the microsatellite motifs of DSI-A-*s* are highlighted in grey. In bold, the parts that are shared by *Sand* s alleles, with the insertion site highlighted in dark grey.

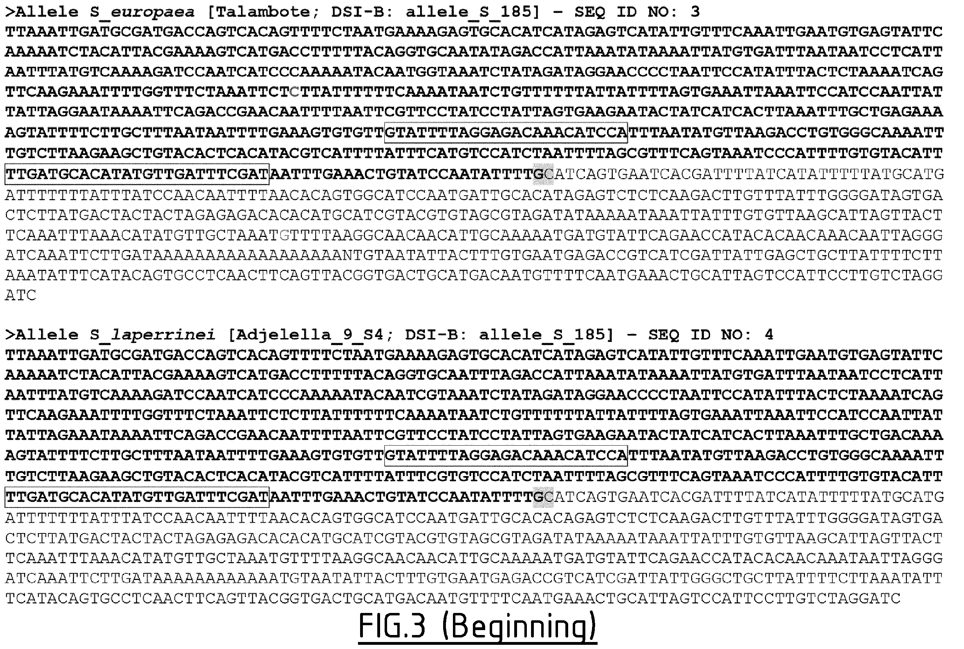

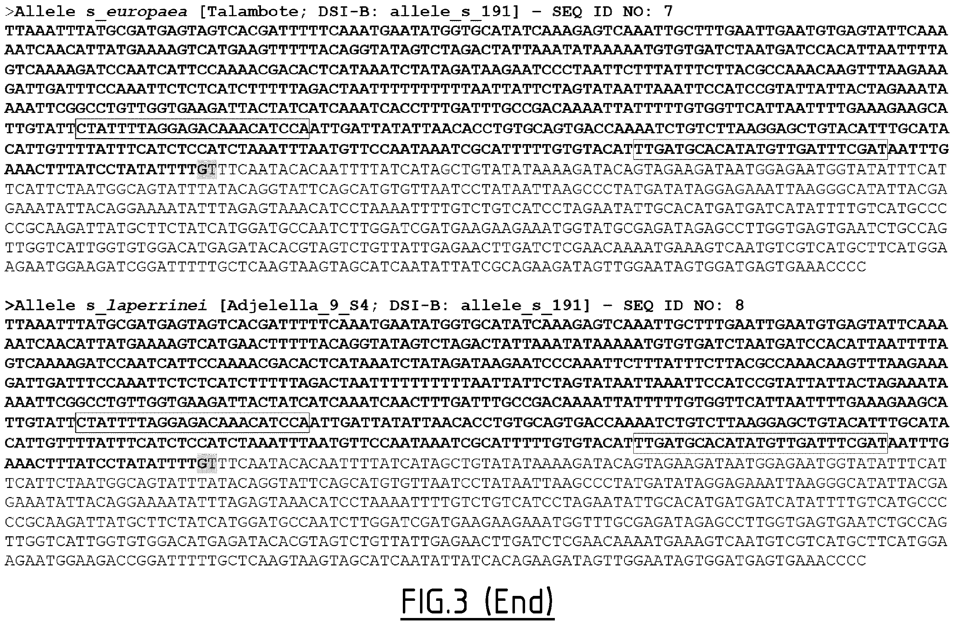

**Figure 3****:** DNA segments of *S* and *s* alleles at the two insertion sites of the hemizygous DSI region [Junction DSI-B]. Sequences were isolated from two G1 individuals belonging to subspecies *europaea* and *laperrinei.* Location of primers is represented by frames, and the microsatellite motifs of DSI-A-*s* are highlighted in grey. In bold, the parts that are shared by *Sand s* alleles, with the insertion site highlighted in dark grey.

### EXAMPLES

### Example 1: Test of the C6030 marker on a few Mediterranean and African olives of the CEFE collection

In a preliminary study, we first tested the DSI marker ('C6030') developed by Mariotti et al. (2020, Front. Plant Sci. 10: 1760) that should allow distinguishing both cross-compatibility groups in olive. We analyzed a few trees of our collection for which cross-compatibility phenotype was determined based on realized matings through parentage analyses from seeds or seedlings (Besnard et al., 2020, Ecol. Evol. 10: 1876-1888).

### Material and methods

Eight genotypes were first tested with the marker 'C6030'. We tested four genotypes of *Olea europaea* subsp. *europaea* (Mediterranean Olive; hereafter "*europaea*") and four genotypes of *O. e.* subsp. *cuspidata* (African olive; hereafter "*cuspidata*")*.* For *europaea,* three G1 and one G2 were selected, while two G1 and two G2 were analyzed on *cuspidata* (Table 1). In addition, a few selfings were available for the four *europaea* individuals and were also characterized with the same protocol to check the inheritance of C6030 alleles; among the 34 self-progenies, 25 were from L4R14 ('Koroneiki'), four from L4R19 ('Cailletier'), two from L4R13 ('Arbequina'), and three from L4R17 (`Verdale de Millas'). The C6030 region was amplified following the protocol described by Mariotti et al. (2020, Front Plant Sci 10:1760); PCR was not successful on some individuals (i.e. all tested accessions of *cuspidata* and nine self-progenies of L4R14). The PCR protocol was slightly modified by decreasing the annealing temperature (Tₐ) to 55°C, which allowed us to get an amplification on all samples. PCR product sequencing (Sanger) was performed at GenoScreen (Lille).

**Table 1. List of accessions analyzed for the C6030 region, their cross-compatibility (CC) group (from Besnard et al., 2020, Ecol. Evol. 10: 1876-1888), their C6030 genotype, and for europaea individuals, those of their self-progenies.**

| **Subspecies** | **Individual** | **CC group** | **C6030 genotype** | **C6030 genotype of self- progenies** |
|---|---|---|---|---|
| *europaea* | L4R19 ('Cailletier') | G1 | S-A/s-c | S-A/s-c (2/4) or S-A (2/4) |
| | L4R13 ('Arbequina') | G1 | S-A/s-b2 | S-A/s-b2 (2/2) |
| | L4R14 ('Koroneiki') | G1 | s-b2 | s-b2 (16/25) or Cm (9/25) |
| | L4R17 (`Verdale de Millas') | G2 | s-b2 | s-b2 (3/3) |
| *cuspidata* | Campbelltown 28 | G1 | C1 | - |
| | Grahamstown 5 | G1 | C1/C2 | - |
| | Campbelltown 27 | G2 | C1 | - |
| | Grahamstown 8 | G2 | C1/C2 | - |

### Results

Among the *europaea* individuals, a novel haplotype was detected in three individuals. This haplotype was named s-b2 [as it was distinguished from s-b by only one single nucleotide polymorphism in the 3' part of the sequence; position 426 according to Mariotti et al. (2020, Front. Plant Sci. 10: 1760)]. It was detected in L4R13 (G1), L4R14 (G1) and L4R17 (G2; Table 1). Unexpectedly, L4R14 (G1) does not show a C6030 profile compatible with a G1 genotype as described in Mariotti et al. (2020, Front. Plant Sci. 10: 1760). Indeed, the apparent homozygous genotype for s-b should be associated to G2 (as observed here on L4R17). The detection of only one haplotype could, however, be due to the presence of null allele as mentioned by Mariotti et al. (2020, Front. Plant Sci. 10: 1760). The analysis of 25 selfings obtained from L4R14 shows that the s-b2 haplotype is not present in nine progenies supporting that a C6030 null allele is indeed present in L4R14. But, another divergent sequence (named Cm) was finally amplified (at Tₐ = 55°C) in these nine progenies. We hypothesized that it may correspond to a paralogous locus that can only be amplified in presence of a homozygous genotype for the null allele at locus C6030. Supporting this hypothesis, the nine selfings for which Cm was observed were finally *SS* according to the DSI markers developed in the present study.

Very divergent sequences were also generated on *cuspidata* individuals and two haplotypes were distinguished (C1 and C2). The combination of these haplotypes does not allow recognizing the cross-compatibility group in the four studied *cuspidata* accessions (Table 1). In addition, the Cm sequence (detected in nine L4R14 progenies) is similar to *cuspidata* sequences suggesting the amplification of a paralogous region, and a lack of specificity of the marker in our PCR conditions (Tₐ = 55°C).

Results of this study do not confirm the expectation that the C6030 profile should allow unambiguously determining the cross-compatibility group of each Mediterranean olive tree (e.g. L4R14; Table 1). In addition, the presence of null alleles [already stated by Mariotti et al. (2020, Front. Plant Sci. 10: 1760)] and/or paralogous sequences should limit the usefulness of this locus in olive, especially at a wide taxonomical scale (i.e. *O. europaea* complex). This calls for the development of a more robust test for determining cross compatibility of olive genotypes.

### Example 2: Identification of a hemizygous supergene that controls homomorphic di-allelic self-incompatibility in olive

We used RAD-sequencing (RAD-seq) of SI-phenotyped individuals from three olive subspecies to detect SI-associated regions. Our analyses led us to revise the genomic location of the *S*-locus in olive as we demonstrated that a hemizygous region is present in only one of the two mating types, suggesting it controls self-incompatibility.

### Material and Methods

### WGS and assembly of the Laperrine's olive genome

High-molecular weight DNA from fresh leaves was extracted for whole genome sequencing of Laperrine's olive individual 'Adjelella 9_S4' (phenotyped as G1, and thus expected to have a *Ss* genotype; Besnard et al., 2020, Ecol. Evol. 10: 1876-1888). Sequencing was performed using two PacBio Hifi SMRT cells. Hi-fi reads were corrected and assembled using HiFiasm (Cheng et al., 2021, Nat. Methods 18: 170-175) which produces partially phased assemblies, i.e. two complete assemblies with long stretches of phased blocks, representing an entire diploid genome. Allele separation was checked via *k*-mer analysis with KAT (Mapleson et al., 2017, Bioinformatics 33: 574-576). All steps from extraction to assembly were performed at the CNRGV (Centre National de Ressources Génomiques Végétales, Toulouse, France). We used Minimap2 (Li et al., 2018, Bioinformatics, 34: 3094-3100) to map back long read on the haplotype assembly and to perform whole-chromosome alignment. Genome assemblies were softmasked using Red (Girgis, 2015, BMC Bioinformatics 16: 227) and annotated using Braker2 (Brůna *et al.,* 2021, *NAR Genomics Bioinformatics* 3: iqaa108) with hints from OrthoDB (Zdobnov et al., 2021, Nucleic Acids Res. 49: D389-D393). We only kept genes at least partially supported by external hints. Functional annotation was performed with InterProScan with default parameters (Jones et al., 2014, Bioinformatics 30: 1236-1240). We used EDTA (Ou et al., 2019, Genome Biol. 20: 275) to identify transposable elements (TE). Syntenic regions between the three olive genome assemblies were identified using MCscan and jcvi python library (Tang et al., 2008, Science 320: 486-488).

### Plant sampling, RAD-sequencing, and reads processing

We selected 37 accessions of three different olive subspecies (12 *O. e.* subsp. *europaea,* 18 *O. e.* subsp. *laperrinei,* and 10 *O. e.* subsp. *cuspidata*) for which the SI phenotype has been previously determined using paternity tests on realized matings (Besnard et al., 2020, Ecol. Evol. 10: 1876-1888). In total, 19 individuals are G1 (*Ss*) and 18 are G2 (*ss*; Table 2). DNAs were extracted with BioSprint (Qiagen), and 200 ng were then digested with *Pst*I*.* RAD-seq libraries were prepared at the GenoToul sequencing platform facility (Toulouse, France) following the protocol described in Etter et al. (2011, Molecular Methods for Evolutionary Genetics. In: Orgogozo V, Rockman M V, editors. Totowa, NJ: Humana Press. p. 157-178). Samples were pooled into three distinct libraries and multiplexed. The three libraries were sequenced on one lane of Illumina NovaSeq run to produce 150-bp paired-end reads. Reads were demultiplexed and cleaned with the process_radtags module of Stacks v2.5 (Rochette et al., 2019, Mol. Ecol. 28: 4737-4754), allowing the rescue of reads with two mismatches in barcodes (less than the distance between any two barcodes in the used set). Cleaned demultiplexed reads were then mapped using Bowtie2 (with default parameters; Langmead and Salzberg, 2012, Nat. Methods 9: 357-359) to the oleaster reference genome (Unver et al., 2017, Proc. Natl. Acad. Sci. USA 114: E9413-E9422), as well as the reference genome for the olive cultivar 'Arbequina' (Rao et al., 2021, Hortic. Res. 8: 64), and finally to the haplotype genome assemblies of the Laperrine's olive generated in this study.

**Table 2. List of plant material analyzed with RAD-sequencing (RAD-seq).**

| **Subsp.** | **Accession name** | **CC¹ group** | **Subsp.** | **Accession name** | **CC group** |
|---|---|---|---|---|---|
| *europaea* | L4R10 ('Zard') | G2 | *laperrinei* | Adjelella 9_S1 | G2 |
| | L4R11 (`Manzanilla') | G2 | | Adjelella 9_S4* | G1 |
| | L4R13 ('Arbequina') | G1 | | Adjelella 10_S9 | G2 |
| | L4R14 ('Koroneiki') | G1 | | Adjelella 10_S10 | G1 |
| | L4R15 | G1 | | Adjelella 10_S11 | G2 |
| | L4R17 ('Verdale de Millas) | G2 | | Adjelella 10_S12 | G1 |
| | L4R19 ('Cailletier') | G1 | | Tin-Hamor S1 | G2 |
| | L4R24 ('Urla 6') | G2 | | Tin-Hamor S4 | G1 |
| | L4R25 ('Al Ascharinah 9') | G2 | | Tin-Hamor S6 | G2 |
| | | | | Tin-Hamor S7 | G2 |
| *cuspidata* | Grahamstown 3 | G1 | | Tin-Hamor S10 | G1 |
| | Grahamstown 5 | G1 | | Tin-Hamor S11 | G1 |
| | Grahamstown 7 | G1 | | Tin-Hamor S14 | G1 |
| | Grahamstown 8 | G2 | | Akerakar 3_S1 | G1 |
| | Campelltown 27 | G2 | | Tonget A | G2 |
| | Campelltown 28 | G1 | | Tonget C | G1 |
| | Campelltown 29 | G2 | | Tonget F | G2 |
| | Kirstenbosch 1 | G2 | | Tonget D | G1 |
| | Kirstenbosch 2 | G2 | | | |
| | Kirstenbosch 4 | G1 | | | |

| | | | | | |
|---|---|---|---|---|---|
| ¹ cross-compatibility (CC) groups were determined in Besnard et al. (2020, Ecol. Evol. 10: 1876-1888); * individual used for whole genome sequencing; subsp. = subspecies. | | | | | |

### Variant calling and population structure

From read alignments on each reference genome, we called bi-allelic SNPs with bcftools (Danecek et al., 2021, GigaScience 10: giab008), keeping only reads with a mapping quality above 20 (excluding multi-mappers), a base-calling quality of at least 20, and a mean read depth over all individuals between 5 and 60 (twice the mean depth to exclude errors due to mapping in paralogous/repetitive regions). We further used vcftools (Danecek et al., 2021, GigaScience 10: giab008) to filter sites missing in more than 90% of individuals and those with a minor allele frequency of 0.05. For an individual genotype to be called, we also required a minimum coverage of 5. We only kept one site every 1000 bp to perform a principal component analysis (PCA) of the 37 samples using vcfR and adegenet packages in R (Jombart and Ahmed, 2011, Bioinformatics 27: 3070-3071; Knaus and Grünwald, 2017, Mol. Ecol. Resour. 17: 44-53).

### Detecting variation in depth of coverage between groups

The two haplotypes may have diverged enough to prevent reads from one haplotype to properly map to the haplotype assembled in the haploid reference, similarly to what is observed for sex chromosomes (Qiu et al., 2016, Mol. Ecol. 25: 414-430; Palmer et al., 2019, Mol. Ecol. 28: 4709-4724). Moreover, hemizygosity is a common feature of several heteromorphic DSI systems and could explain the absence of recombination permitting the conservation of the DSI system over a long evolutionary period. Both configurations would result in sequencing-depth variation between groups. The cross-compatibility phenotype of the individual sequenced for the oleaster reference genome is unknown. Both cultivar 'Arbequina' (Rao et al., 2021, Hortic. Res. 8: 64) and the Laperrine's individual we used for whole-genome sequencing are G1 (Besnard et al., 2020, Ecol. Evol. 10: 1876-1888), and thus expected to be *Ss.* If the assembled haplotype is s, the *Ss*:*ss* ratio would be close to 0.5 at the *S*-locus. Conversely, if the assembled haplotype is *S*, the *Ss*:*ss* ratio would be infinite. We used SeqKit (Shen et al., 2016, PLoS One 11: e0163962) to predict *Pst*I restriction sites in the three different reference genomes to which reads were mapped. We then estimated the depth of coverage at each predicted *Pst*I locus with samtools (Danecek et al., 2021, GigaScience 10: giab008). For each sample, we normalized the counts by dividing by the total number of mapped reads. We filtered out sites, removing those with a median normalized coverage smaller than 0.5 read per million of mapped reads across both groups. The ratio between the median depth in *Ss* individuals and *ss* individuals, calculated as log2[(*Ss*+1)/(*ss*+1)], along the genome was plotted by 100-kb sliding-windows with a step size of 25 kb, using the python library matplotlib (Hunter, 2007, Comp. Sci. Eng. 9: 90-95). We also examined the occurrence of a significant depth difference between Ss and ss individuals in 50 kb windows along each reference genome using two-sided Wilcoxon tests. To increase our power and as we are looking for a genetic region conserved across species, we pooled individuals from each incompatibility group together, regardless of the olive subspecies.

### Results

### High-quality haplotype assemblies of the Saharan olive genome

The two SMRT cells produced 3,659,295 reads with a median size of 18.75 kb. The *k*-mer model estimated a coverage of 48× for a haploid genome size of 1.3 Gb. Hifiasm produced two partially phased assemblies (or haplotypes). Overall, 68,607 and 65,939 protein-coding genes were annotated in haplotypes 1 and 2, respectively, with at least partial support from protein hints. Synteny analyses identified 24 scaffolds collinear with the 23 chromosomes of the previously published assembly in haplotype 1, as chromosome 18 is split between two scaffolds in our assembly. For haplotype 2, we retrieved 28 scaffolds that match the original 23 chromosomes.

### RAD-seq reveals a large peak of differential coverage between incompatibility groups

Across the 37 olive samples, a total of 618,291,848 paired end 150-bp reads were generated, 99% of which were retained after quality filtering and demultiplexing. The mapping rate over the Laperrine's olive genome assembly ranged from 84 to 93.5%. Four reference genomes (i.e. oleaster, 'Arbequina' and the two haploid genomes of the Laperrine's olive) were then scanned for variation in coverage ratio between the two incompatibility groups for each subspecies. On chromosome 18 (of the oleaster reference genome), an increase of the *Ss*:*ss* coverage ratio was observed between 16.7 and 16.9 Mb in the three subspecies. Our Wilcoxon tests for difference in depth between groups (individuals pooled by incompatibility groups regardless of the species), indeed identified three windows at these coordinates as significantly more covered in Ss individuals (FDR < 0.05). Three more windows with this profile were detected on two unanchored scaffolds: NW_019268110.1 (from 0.5 to 1 kb over a total size of 262 kb) and NW_019238463 (0.5 to 1 kb for a total size of 145 kb). We did not detect any windows shared across the three subspecies with the opposite profile (higher coverage in ss than in *Ss*). Similar results are observed using as reference the olive cultivar 'Arbequina'. A unique and large increase in coverage ratio is observed on chromosome 18 in the three subspecies between 18.7 and 19.5 Mb. This is supported by Wilcoxon tests results, as only nine 50-kb windows have significant difference in depth, all between 18.85 and 19.35 Mb. In Laperrine's olive haplotype assemblies, we detected a large peak in 'haplotype 1' between 1.6 and 2.3 Mb. All 50-kb windows between 1.6 and 2.3 Mb were significant for a different depth and were the only significant windows in this assembly. As a consequence, we suggest the assembled haplotype in both reference genome as well as in our 'haplotype 1' assembly is the dominant *S* haplotype. On the contrary, we did not detect any peak or significant window (FDR < 0.05) in any direction in 'haplotype 2', that we infer to be the recessive s haplotype. It means that individuals from both incompatibility types were sequenced and mapped with similar rates on this haplotype assembly. This last observation would only be compatible with a moderate divergence between the two haplotypes at the S-locus or with its absence. As our other observations reject the former, we postulate the dominant S haplotype is hemizygous.

### A single sequence insertion differentiates the two incompatibility types

Comparison of the scaffolds corresponding to chromosome 18 in the two Laperrine's haplotype assemblies revealed a large indel of over 700 kb underlying the coverage variation between the two groups. This region in 'haplotype 1' has no homolog in 'haplotype 2', yet the flanking regions have homologs in 'haplotype 2', where they are adjacent. These patterns indicate that a 0.7 Mb-stretch of DNA is present in 'haplotype 1', yet missing from 'haplotype 2'. We confirmed the existence of this insertion by mapping back HiFi reads on the two haplotype-resolved assemblies. Inspection of the mapped reads at this location confirmed the presence of whole reads covering the junction without the indel (on 'haplotype 2') and other reads including the indel sequence and some downstream/upstream sequence (on 'haplotype 1').

Synteny analysis confirmed that the sequence underlying the large peak observed around 19 Mb in the 'Arbequina' genome is syntenic to the 'haplotype 1'-specific sequence. According to RAD-seq data, this region is totally absent in *ss* individuals, regardless of the subspecies, supporting our conclusion that this sequence is the *S* haplotype. Compared to 'haplotype 1' Laperrine's sequence, a deletion of 50 kb within this region is observed in 'Arbequina' (confirmed here by our RAD-seq data from this cultivar), which also presents an inversion in the oleaster genome (Unver et al., 2017, Proc. Natl. Acad. Sci. USA 114: E9413-E9422). In the oleaster assembly, the sequences behind the peak detected on chromosome 18 (at 16.8 Mb) as well as on the two unanchored scaffolds are also syntenic fragments of the 0.7-Mb *S*-specific region. The full *S*-locus is thus present but scattered across this genome assembly [in chromosome 18 (5' end), plus unanchored scaffolds NW_019238463.1 (middle) and NW_019268110.1 (3' end, with some downstream sequence)]. The newly identified region is, in both 'haplotype 1' and 'Arbequina' assemblies, contiguous to the candidate region defined by Mariotti et al. (2020, Front. Plant Sci. 10: 1760), but 8 Mb apart in the oleaster assembly.

We annotated 17 genes in the 0.7-Mb *S*-specific region of the Laperrine's olive, most of them of unknown functions (Table 3). Five LTRs were identified by EDTA within the region, as well as two MITEs. In addition, four of our predicted protein-coding genes had TE-related functions. Finally, four annotated genes were common in the Laperrine's olive, oleaster, and 'Arbequina' genome annotations. Two of them seem particularly interesting due to their annotation suggesting functions related to hormone regulation: g33223.t1 is annotated as a *bzr (BRASSINAZOLE-RESISTANT)* gene homolog, and g33233.t1 as a gibberellin 2-beta-dioxygenase homolog. We also note the presence in our annotation of a second BES/BZR homolog upstream of this one, but solely supported by computational prediction with no biological hints. This gene was present in the two other genome annotations.

**Table 3. Predicted function of S-locus genes and correspondence between genome annotation.**

| **Gene ID in 'haplotype** | **1' Gene ID in 'Arbequina'** | **Gene ID in oleaster** | **Prediction about function** |
|---|---|---|---|
| g33207.t1 | - | - | - |
| g33208.t1 | - | - | - |
| g33210.t1 | GWHTAOPM038200 | LOC111366801 | - |
| g33212.t1 | NA¹ | - | - |
| g33216.t1 | - | - | TE-related |
| g33217.t2 | - | - | - |
| g33218.t1 | - | - | - |
| g33219.t1 | - | - | IPP transferase |
| g33222.t1 | - | - | TE-related |
| g33223.t1 | GWHPAOPM038191 | LOC111379898 | BES1/BZR1 plant transcription factor |
| g33228.t1 | - | - | - |
| g33230.t1 | - | - | - |
| g33231.t1 | GWHPAOPM038189 | LOC111392691 | Aminotransferase-like, PLP-dependent enzymes |
| g33232.t1 | - | - | TE-related |
| g33233.t1 | GWHPAOPM038188 | LOC111392689 | Gibberellin 2-beta-dioxygenase 2 |
| g33235.t1 | - | - | TE-related |
| g33236.t2 | - | - | Exostosin heparan sulfate glycosyltransferase |

| | | | |
|---|---|---|---|
| ¹ Corresponding sequence is within a deletion in this cultivar. | | | |

### Example 3: Development of a simple PCR-based diagnosis of cross-compatibility groups in olive (Olea europaea L.)

Developing a robust PCR test of the DSI locus (i.e. useful on any olive accession, and able to simultaneously amplify a segment specific to *s* and *S* alleles) requires comparing the most genetically divergent *O. europaea* accessions in order to define primers in conserved sequences. Our test was thus developed and validated on trees coming from three continents (from South Africa to Asia, the Central Sahara and the Mediterranean Basin). We also characterized self-progenies of G1 cultivated olives to test the occurrence and viability of SS individuals.

### Material and methods

### Plant material

Olive belongs to a species complex (the so-called *O. europaea* complex) that diversified since the Late Miocene, about 6 to 8 Mya (Green, 2002, Kew Bull. 57: 91-140; Besnard et al., 2009, Ann. Bot. 104: 143-160). All wild olives are native to the Old World (from the Mediterranean Basin, the Canary and Madeira archipelagos, Tropical Africa and southern Asia; Green 2002, Kew Bull. 57: 91-140). In the collection at the common garden of the "Plateforme des Terrains d'Experience du LabEx CeMEB," (CEFE, CNRS, Montpellier, France), we currently maintained more than 100 olive accessions belonging to subspecies *europaea* (Mediterranean olive), *laperrinei* (Laperrine's olive), *cuspidata* (African olive) and *maroccana* (Moroccan olive, hexaploid). Sixty-nine trees were phenotyped for their cross-compatibility group (G1 or G2) and retained for this study. They include 38 individuals of *laperrinei,* 16 *europaea* (i.e. 12 cultivars and four oleasters), eleven *cuspidata,* and four hybrids. Thirty trees are G1, while the 39 remaining are G2. In addition, the 34 self-progenies mentioned above were also characterized with our DSI markers, in order to test the presence of *SS* individuals in G1 progenies [issued from L4R19 ('Cailletier'), L4R13 ('Arbequina') or L4R14 ('Koroneiki')].

### Designing of primers to genotype the DSI locus

After aligning the two 18-chromosome haplocontigs of the G1 Laperrine's olive (h2tg000033I and h1tg000037I), extremities of the hemizygous region were located (positions 1,658,780 and 2,393,593 in halpocontig h1tg000037I). On each extremity, a DNA segment (ca. 700-1200 bp) of both *S* and *s* alleles covering the insertion site of the hemizygous region were isolated and then used as references (full sequences of *europaea* and *laperrinei* are given in Figures 2-3). HiSeq reads genome skimming data available for two olive G1 accessions of *europaea* and *cuspidata* were mapped on these two alleles in order to compare these regions among distantly related olive accessions. Three primers were first defined in short conserved regions of *S* and/or *s* alleles at the 5' extremity (DSI-A; Table 4; Figures 1-2). Primer S/s_A_For (including a M13 tail in 5') was defined in a conserved region on both S and s alleles (less than 40 bp before the insertion of the hemizygous region). In contrast, one reverse primer was defined specifically for each *s* and S alleles - s_A(1)_Rev and S_A_Rev - the latter being located in the hemizygous region. Expected size of PCR products was 441-451 and 298-333 bp for the s and S alleles, respectively. Length variation was due to a 4-bp-microsatellite (TTAT motif) and a 10-bp indel in the s allele, while a 35-bp indel was observed in the S alleles between *europaea* and *cuspidata*/*laperrinei.* In addition, we defined an alternative reverse primer to amplify a shorter segment of alleles *s*: s_A(2)_Rev. This primer can be used instead of s_A(1)_Rev to generate a s fragment of 186 bp in all studied olive subspecies. On the 3' extremity (DSI-B), we then used the same approach and designed two primers [S/s_B_For (including a M13 tail in 5') and S/s_B_Rev] to amplify a segment of 190-191 and 185 bp for the *s* and S alleles, respectively (Table 4). Finally, we amplified specifically a short segment of the g33233.t1gene (Table 4), which is unique to the *S*-supergene. Primers were designed in a region conserved between homologs of distantly related species [(i.e. *O. europaea* (Oleeae) and *C. fruticans* (Jasmineae)], while as divergent as possible among their olive paralogs.

**Table 4: Primers used to amplify extremities of the DSI locus (DSI_A and DSI_B), and a segment of the g33233 gene. PCR product size is given in bp for Sand s haplotypes of the S locus (see Table SM2 for genotypic profiles).**

| | | | |
|---|---|---|---|
| **Locus** | **For (5' → 3')** | **Rev (5' → 3')** | **Allele size (bp)** |
| DSI-A(1) | S/s_A: | s A(1): | *S* = 298, 332; |
| | GAACAAACAATGAGCTTGTAT TTC (SEQ ID NO:9) | [?] CCCAACAATTCCACGTTGCT (SEQ ID NO:11) | |
| | or | S_A: | *s* = 438, 442, 446, 452 |
| | tgtaaaacgacggccagtGAACAAA CAATGAGCTTGTATTTC (SEQ ID NO:10) | TGGTAAAAGGATATCATTGT TACC (SEQ ID NO:12) | |
| DSI-A(2) | S/s_A: | s_A(2): | *S* = 332; *s* = 186 |
| | GAACAAACAATGAGCTTGTAT TTC (SEQ ID NO:9) | TTACCCTAGTACATTAACTT TCCA (SEQ ID NO:13) | |
| | or | S_A: | |
| | tgtaaaacgacggccagtGAACAAA CAATGAGCTTGTATTTC (SEQ ID NO:10) | TGGTAAAAGGATATCATTGT TACC (SEQ ID NO:12) | |
| DSI-B | S/s_B: | S/s_B: | *S* = 185; *s* = 190, 191 |
| | STATTTTAGGAGACAAACATC CA (SEQ ID NO:14) | | |
| | or | ATCGAAATCAACATATGTGC ATCAA (SEQ ID NO: 16) | |
| | tgtaaaacgacggccagtSTATTTTA GGAGACAAACATCCA (SEQ ID NO: 15) | | |
| g33233 | TGCAAGCCCATTTCAASCAYY TTC (SEQ ID NO: 17) | CTTAWTGGCTTCACATTCCA ATCTA (SEQ ID NO: 18) | *S* = 168; *s* = null |

### Laboratory procedures

For DSI-A and DSI-B, *S* and s alleles of the DSI locus were amplified by PCR following the method described by Schuelke (2000, Nat. Biotechnol. 18: 233-234). Each DSI-A PCR reaction (25 µL) contained 10 ng DNA template, 1× reaction buffer, 3.5 mM MgCl₂, 0.2 mM dNTPs, 0.1 µmol of 6-FAM-labeled M13(-21) primer (^{5'}TGTAAAACGACGGCCAGT^{3'}), 0.1 µmol of the S/s_A_For primer, 0.1 µmol of each reverse primer [preferentially s_A(1)_Rev and S_A_Rev to reveal different s alleles, or alternatively s_A(2)_Rev and S_A_Rev that generate a unique shorter s allele (Table 4)], and 0.5 U of *Taq* DNA polymerase (Promega). For DSI-B, we used a similar PCR protocol except for primers: 0.1 µmol of HEX-labelled M13(-21) primer, 0.1 µmol of the S/s_B_For primer, and 0.2 µmol of the S/s_B_Rev primer (Table 4). We conducted PCR of both loci in a Mastercycler pro PCR System (Eppendorf) for 2 min at 94°C, followed by 25 cycles of 30 s at 94°C, 45 s at 56°C, and 1 min at 72°C, and then by 10 cycles of 30 s at 94°C, 45 s at 51.5°C, and 45 s at 72°C. The last cycle was followed by a 20-min extension at 72°C. PCR products were finally multiplexed with GenScan-500ROX (Applied Biosystems) and separated on an ABI Prism 3730 DNA Analyzer (Applied Biosystems). Chromatograms were read with Geneious v.9 (Kearse et al., 2012, Bioinformatics 28: 1647-1649).

Furthermore, the g33233 locus was amplified following the next procedure: Each PCR reaction (25 µL) contained 10 ng DNA template, 1× reaction buffer, 2.5 mM MgCl₂, 0.2 mM dNTPs, 0.2 µmol of each primer (Table 4), and 0.5 U of *Taq* DNA polymerase (Promega). We conducted PCR in a Mastercycler pro PCR System (Eppendorf) for 2 min at 94°C, followed by 35 cycles of 30 s at 94°C, 45 s at 56°C, and 1 min at 72°C. The last cycle was followed by a 20-min extension at 72°C. PCR products were then migrated on a 2%-agarose gel. For this marker, presence or absence of amplification was scored.

### Results and discussion

The three targeted regions were all successfully amplified and generated *S and* s alleles at the expected size. Interestingly, different *s* size variants were revealed on DSI-1(A) (4 alleles) and DSI-B (2 alleles; Table 5). For the *S*-haplotype, DSI-A(1)_298 (*europaea*) or _332 (*laperrinei-cuspidata*)*,* DSI-B_185, and g33233_168 are revealed only in G1 individuals (Table 5). As being co-dominant and easy to amplify and score, the use of DSI-B is particularly suitable for a fast, reliable characterization of cross-compatibility groups in olive germplasm collections.

**Table 5: Genotypic profiles at three loci of the DSI region observed in three olive subspecies and hybrids (i.e. 69 trees from the CEFE collection), and their associated cross-compatibility (CC) group. As expected for the S-haplotype, DSI-A(1)_298/332, DSI-B_185, and g33233_168 (all in bold and underlined), co-segregate and are revealed only in G1 individuals. N = Number of trees.**

| **Taxon** | **CC group** | **DSI genotypes** | | | **N** |
|---|---|---|---|---|---|
| | | **DSI_A(1)** | **g33233** | **DSI_B** | |
| *europaea* (16) | G1 | **298**:452 | **168** | **185**:190 | 4 |
| | G2 | 452:452 | 0 | 190:190 | 8 |
| | | 446:446 | 0 | 191:191 | 2 |
| | | 446:452 | 0 | 190:191 | 1 |
| | | 438:452 | 0 | 190:191 | 1 |
| | | | | | |
| *laperrinei* (38) | G1 | **332**:442 | **168** | **185**:191 | 19 |
| | G2 | 442:442 | 0 | 191:191 | 19 |
| | | | | | |
| *cuspidata* (11) | G1 | **332**:442 | **168** | **185**:190 | 5 |
| | G2 | 442:442 | 0 | 190:190 | 6 |
| | | | | | |
| Hybrids (4) | G1 | **298**:452 | **168** | **185**:190 | 1 |
| | | **332**:452 | **168** | **185:**190 | 1 |
| | G2 | 442:442 | 0 | 191:191 | 1 |
| | | 442:452 | 0 | 190:191 | 1 |

## Claims

1. A method for determining the self-incompatibility genotype of an olive tree comprising:
c) detecting the presence (allele *S*) or absence (allele *s*) of a 0.7 Mb insertion-deletion (indel) sequence in its chromosomes 18;
d) determining the self-incompatibility genotype of the olive tree as being either *Ss* if the 0.7-Mb indel sequence is present at the hemizygous state in one chromosome 18, or *ss* if the 0.7-Mb indel sequence is absent in both chromosomes 18;
wherein allele *S* is dominant over allele s, and genotypes *Ss* and *ss* constitute the two inter-compatible genotypic combinations of the diallelic self-incompatibility (DSI) system in olive tree.

2. A method for determining the self-incompatibility phenotype of an olive tree comprising:
c) detecting the presence (allele *S*) or absence (allele *s*) of a 0.7 Mb insertion-deletion (indel) sequence in its chromosomes 18;
d) determining the self-incompatibility phenotype of the olive tree as being either self-incompatibility group G1 if the 0.7-Mb indel sequence is present at the hemizygous state in one chromosome 18, or self-incompatibility group G2 if the 0.7-Mb indel sequence is absent in both chromosomes 18;
wherein olive trees from group G1 cannot cross-fertilize each others, and olive trees from group G2 cannot cross-fertilize each others.

3. The method according to claim 1 or 2, wherein the 0.7-Mb indel sequence in chromosome 18 of the olive tree comprises nucleotides from position 100,501 to position 835,222 of sequence SEQ ID NO:19, or a sequence at least 75% identical to SEQ ID NO:19.

4. The method according to any one of claims 1 to 3, wherein detecting the presence, at the hemizygous state, of the 0.7-Mb indel sequence in one chromosome 18 of the olive tree is performed by:
i) Amplifying a region of at least 12 contiguous nucleotides of chromosome 18 that comprises at least nucleotides at positions 422-423 of SEQ ID NO: 1 or SEQ ID NO:2, or
ii) Amplifying a region of chromosome 18 that comprises nucleotides at positions 505-623 of SEQ ID NO: 3 or SEQ ID NO: 4; or
iii) detecting presence of g33233.t1 gene in G1 individuals, wherein g33233.t1 gene comprises positions 668762 to 670268 of SEQ ID NO:19, or a sequence at least 75% identical thereto.

5. The method according to any one of claims 1 to 4, wherein detecting the absence of the 0.7-Mb indel sequence in chromosome 18 of the olive tree is performed by detecting absence of g33233.t1 gene.

6. The method according to any one of claims 1 to 5, wherein:
a) detecting the presence, at the hemizygous state, or absence of a 0.7-Mb indel sequence in chromosomes 18 of an olive tree is performed by PCR amplification using a forward primer comprising sequence SEQ ID NO:9, a first reverse primer comprising sequence SEQ ID NO:11, and a second reverse primer comprising sequence SEQ ID NO:12 or sequence SEQ ID NO:13;
b) identifying the olive tree as having a genotype *Ss,* or phenotype G1, if the size of one of the amplicons is in the range 290 to 340 bp, or identifying the olive tree as having a genotype ss, or phenotype G2, if the size of the amplicons is only 186 bp when the pair of reverse primers comprises SEQ ID NO: 11 and SEQ ID NO: 13, or only in the range 430 to 460 bp when the pair of reverse primers comprises SEQ ID NO: 11 and SEQ ID NO:12.

7. The method according to any one of claims 1 to 6, wherein:
a) detecting the presence, at the hemizygous state, or absence of a 0.7-Mb indel sequence in chromosomes 18 of an olive tree is performed by PCR amplification using a forward primer comprising sequence SEQ ID NO:14 and a reverse primer comprising sequence SEQ ID NO:16;
b) identifying the olive tree as having a genotype *Ss,* or phenotype G1, if the size of one of the amplicons is 185 bp, or identifying the olive tree as having a genotype *ss,* or phenotype G2, if the size of the amplicons is only at 190-191 bp.

8. The method according to any one of claims 1 to 7, wherein:
a) detecting the presence, at the hemizygous state, or absence of a 0.7-Mb indel sequence in chromosomes 18 of an olive tree is performed by PCR amplification using a forward primer comprising sequence SEQ ID NO:17 and a reverse primer comprising sequence SEQ ID NO:18;
b) identifying the olive tree as having a genotype *Ss,* or phenotype G1, if the size an amplicon is 168 bp, or identifying the olive tree as having a genotype ss, or phenotype G2, if the PCR amplification produces no amplicon.

9. A method for determining cross-compatibility between at least two olive trees, comprising:
a) determining the self-incompatibility phenotype or genotype of at least two olive trees by a method according to any one of claims 1 to 8;
b) identifying the at least two olive trees as cross-compatible if they have different self-incompatibility phenotypes (G1 and G2) or genotypes (*Ss* and ss), and identifying the two olive trees as cross-incompatible if they have the same self-incompatibility phenotype (either G1 and G1, or G2 and G2) or genotype (either *Ss* and *Ss,* or ss and ss).

10. A method of planting olive trees in an orchard comprising:
a) determining the self-incompatibility genotype or phenotype of olive trees by a method according to any one of claims 1 to 8;
b) selecting olive trees of the G1 and G2 phenotypes, or olive trees of the *Ss* and ss genotypes;
c) planting olive trees in the orchard with a spatial repartition of olive trees defined based on the G1 and G2 phenotypes, or the *Ss* and *ss* genotypes of the olive trees.

11. A method of optimizing olive production comprising:
a) planting olive trees in an orchard by a method according to claim 10;
b) cultivating the planted olive trees under conditions and for a time sufficient for the olive trees to produce olives, and harvesting olives on the cultivated olive trees.

12. The method according to any one of claims 1-11, wherein the olive tree is *Olea europaea* subsp. *europaea* (Mediterranean olive), *O. e.* subsp. *laperrinei* (Laperrine's olive), or *O. e.* subsp. *cuspidata* (African olive), or a hybrid thereof.

13. A kit comprising:
i) A forward primer comprising sequence SEQ ID NO:9, a first reverse primer comprising sequence SEQ ID NO:11, and a second reverse primer comprising sequence SEQ ID NO:12 or sequence SEQ ID NO:13;
ii) A forward primer comprising sequence SEQ ID NO:14 and a reverse primer comprising sequence SEQ ID NO:16; or
iii) A forward primer comprising sequence SEQ ID NO:17 and a reverse primer comprising sequence SEQ ID NO:18.

14. A method of performing PCR amplification, comprising:
a) Hybridizing a sample of genomic DNA of olive tree with a set of primers comprising:
[1] A forward primer comprising sequence SEQ ID NO:9, a first reverse primer comprising sequence SEQ ID NO:11, and a second reverse primer comprising sequence SEQ ID NO:12 or sequence SEQ ID NO:13;
[2] A forward primer comprising sequence SEQ ID NO:14 and a reverse primer comprising sequence SEQ ID NO:16; or
[3] A forward primer comprising sequence SEQ ID NO:17 and a reverse primer comprising sequence SEQ ID NO:18; and
b) Performing PCR amplification of said genomic DNA.

15. The method according to claim 14, wherein the sample of genomic DNA of olive tree comprises genomic DNA of chromosomes 18, or of a fragment thereof that includes at least positions 100,501 to 835,222 of sequence SEQ ID NO:19, or a sequence at least 75% identical to positions 100,501 to 835,222 of SEQ ID NO:19.
